(19) Europäisches Patentamt
European Patent Office
Office européen des brevets

(11) Veröffentlichungsnummer: **0 216 105 A1**

(12) **EUROPÄISCHE PATENTANMELDUNG**

(21) Anmeldenummer: 86111105.2

(22) Anmeldetag: 12.08.86

(51) Int.Cl.⁴: C 07 F 9/65
A 01 N 57/36, A 61 K 31/675

(30) Priorität: 23.08.85 DE 3530146

(43) Veröffentlichungstag der Anmeldung: 01.04.87 Patentblatt 87/14

(84) Benannte Vertragsstaaten: AT BE CH DE FR GB IT LI NL

(71) Anmelder: BAYER AG
Konzernverwaltung RP Patentabteilung
D-5090 Leverkusen 1 Bayerwerk(DE)

(72) Erfinder: Bonse, Gerhard, Dr.
Wolfskaul 3
D-5000 Köln 80(DE)

(72) Erfinder: Müller, Nikolaus, Dr.
Knipprather Strasse 98
D-4019 Monheim(DE)

(72) Erfinder: Andrews, Peter, Dr.
Gellertweg 2
D-5600 Wuppertal(DE)

(72) Erfinder: Stendel, Wilhelm, Dr.
In den Birken 55
D-5600 Wuppertal(DE)

(72) Erfinder: Steffens, Robert, Dr.
Roggendorfstrasse 63
D.-5000 Köln 80(DE)

(72) Erfinder: Behrenz, Wolfgang, Dr.
Untergründemich 14
D-5063 Overath(DE)

(54) **Diazaphosphorine, Verfahren zu ihrer Herstellung und ihre Verwendung als Schädlingsbekämpfungsmittel.**

(57) Diazaphosphorine der Formel I

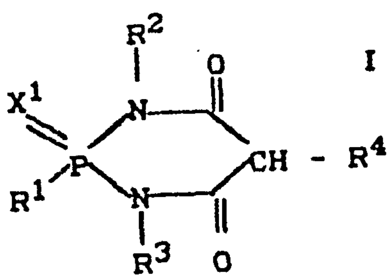

I

in welcher die Substituenten die in der Beschreibung angegebene Bedeutung haben, sowie Verfahren zu ihrer Herstellung.

Die Verbindungen der Formel I lassen sich hervorragend als Schädlingsbekämpfungsmittel, insbesondere als Insektizide und Akarizide auf den Gebieten Land- und Forstwirtschaft, Haushalt, Hygiene, Vorrats- und Materialschutz sowie als Ekto- und Endoparasitizide auf dem Gebiet der Veterinärmedizin einsetzen.

EP 0 216 105 A1

0216105

BAYER AKTIENGESELLSCHAFT
Konzernverwaltung RP
Patentabteilung
5090 Leverkusen, Bayerwerk

Rt/m-c

I

# Diazaphosphorine, Verfahren zu ihrer Herstellung und ihre Verwendung als Schädlingsbekämpfungsmittel

Die vorliegende Erfindung betrifft neue Diazaphosphorine, Verfahren zu ihrer Herstellung, ihre Verwendung als Schädlingsbekämpfungsmittel in Land- und Forstwirtschaft sowie in den Gebieten Haushalt, Hygiene, Vorrats- und Materialschutz und Veterinärmedizin.

Phenylcarbamoylsubstituierte Diazaphosphorine sind bereits bekannt geworden. Sie eignen sich als Schädlingsbekämpfungsmittel sowie als Ektoparasitizide (CH-PS 643 563). Ihre Wirkung, vor allem bei niedrigen Aufwandkonzentrationen, ist jedoch nicht immer voll befriedigend.

Es wurden nun die neuen Diazaphosphorine der Formel I gefunden

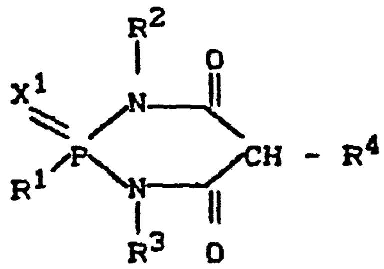

I

in welcher

$X^1$ für O oder S steht

$R^1$ für Aryloxy oder Arylthio steht,

$R^2$ für Wasserstoff, Alkyl, Aryl oder Aralkyl steht,

$R^3$ für Wasserstoff, Alkyl, Aryl oder Aralkyl steht,

$R^4$ für den Rest der Formel steht,

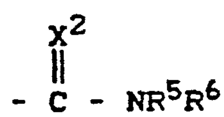

wobei

$X^2$ für O oder S steht,

$R^5$ für Wasserstoff oder Alkyl steht,

$R^6$ für den Fall, daß $X^1$ für O steht, für Phenyl steht, das mindestens einen Substituenten aus der Gruppe $NO_2$, Halogenalkyl, Alkoxy, Phenyloxy, das gegebenenfalls substituiert ist, Alkylthio, Phenylthio, das gegebenenfalls substituiert ist, Halogenalkoxy, Halogenalkylthio, Alkylendioxy, das gegebenenfalls halogensubstituiert ist, Alkylsulfenyl, Alkylsulfonyl, Halogenalkylsulfenyl, Halogenalkylsulfonyl, Halogensulfonyl ($HalSO_2$-) und gegebenenfalls noch weitere Substituenten aus der Gruppe Halogen, Alkyl enthält, unter der Voraussetzung, daß für den Fall, daß $R^6$ für Phenyl steht, das einen Halogenalkylrest trägt, noch mindestens ein weiterer Substituent im Phenylring vorhanden ist, ferner

$R^6$ für den Fall, daß $X^2$ für S steht, für gegebenenfalls substituiertes Phenyl steht.

Die Verbindungen der Formel I können in Form ihrer verschiedenen Tautomeren (Keto/Enol) sowie als Gemische dieser Tautomeren, sowie in Form ihrer Salze mit Basen vorliegen.

Es wurde gefunden, daß man die Verbindungen der Formel I

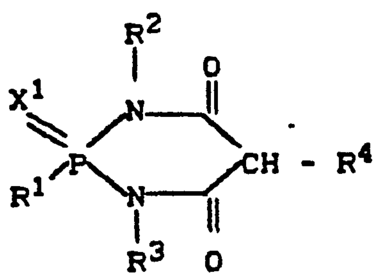

I

in welcher

$X^1$ für O oder S steht

$R^1$ für Aryloxy oder Arylthio steht

$R^2$ für Wasserstoff, Alkyl, Aryl oder Aralkyl steht,

$R^3$ für Wasserstoff, Alkyl, Aryl oder Aralkyl steht,

$R^4$ für den Rest der Formel steht,

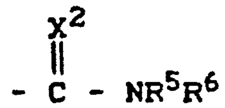

wobei

$X^2$ für O oder S steht,

$R^5$ für Wasserstoff oder Alkyl steht,

$R^6$ für den Fall, daß $X^1$ für O steht, für Phenyl steht, das mindestens einen Substituenten aus der Gruppe $NO_2$, Halogenalkyl, Alkoxy, Phenyloxy, das gegebenenfalls substituiert ist, Alkylthio, Phenylthio, das gegebenenfalls substituiert ist, Halogenalkoxy, Halogenalkylthio, Alkylendioxy, das gegebenenfalls halogensubstituiert ist, Alkylsulfenyl, Alkylsulfonyl, Halogenalkylsulfenyl, Halogenalkylsulfonyl, Halogensulfonyl ($HalSO_2$-) und gegebenenfalls noch weitere Substituenten aus der Gruppe Halogen, Alkyl enthält, unter der Voraussetzung, daß für den Fall, daß $R^6$ für einen Halogenalkylrest steht, noch weitere Substituenten vorhanden sind,

$R^6$ für den Fall, daß $X^2$ für S steht, für gegebenenfalls substituiertes Phenyl steht,

erhält, indem man

a) für den Fall, daß $R^5$ für Wasserstoff steht, Diazaphosphorine der Formel II

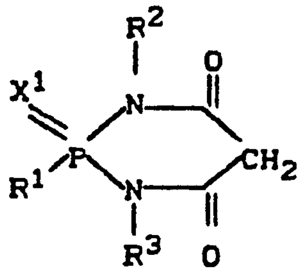

II

in welcher

$X^1$, $R^1$, $R^2$, $R^3$ die oben angegebene Bedeutung haben,

mit Isocyanaten der Formel III

$R^6$ - NCO(S) III

in welcher

$R^6$ die oben angegebene Bedeutung hat,

gegebenenfalls in Gegenwart von Katalysatoren umsetzt,

b) Diazaphosphorine der Formel IV

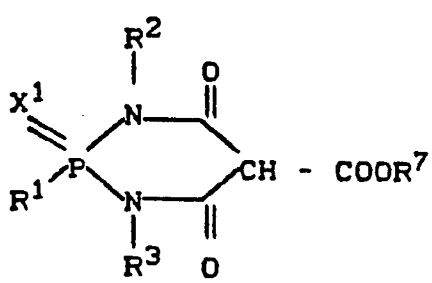

IV

in welcher

$X^1$, $R^1$, $R^2$, $R^3$ die oben angegebene Bedeutung haben,

$R^7$ für $C_1$-$C_4$-Alkyl steht,

mit Aminen der Formel V

$HNR^5R^6$ V

in welcher

$R^5$ und $R^6$ die oben angegebene Bedeutung haben,

umsetzt.

Die Verbindungen der Formel I lassen sich hervorragend als Schädlingsbekämpfungsmittel, insbesondere als Insektizide, Fungizide und Akarizide auf den Gebieten Land- und Forstwirtschaft, Haushalt, Hygiene, Vorrats- und Materialschutz sowie als Ekto- und Endoparasitizide auf dem Gebiet der Veterinärmedizin einsetzen.

Bevorzugt sind Verbindungen der Formel I, in welcher

$X^1$ für O oder S steht, insbesondere für O steht,

$R^1$ für Phenoxy oder Phenylthio, insbesondere Phenoxy, steht, die ein- oder mehrfach, gleich oder verschieden, durch folgende Substituenten substituiert sein können:

Alkyl mit vorzugsweise 1 bis 4, insbesondere 1 oder 2 Kohlenstoffatomen, wie Methyl, Ethyl, n.- und i.-Propyl und n.-, i.- und t.-Butyl; Alkoxy mit vorzugsweise 1 bis 4, insbesondere 1 oder 2 Kohlenstoffatomen, wie Methoxy, Ethoxy, n.- und i.-Propyloxy und n.-, i.- und t.-Butyloxy; Alkylthio mit vorzugsweise 1 bis 4, insbesondere 1 oder 2 Kohlenstoffatomen, wie Methylthio, Ethylthio, n.- und i.-Propylthio und n.-, i.- und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Fluor-, Chlorethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylthio; Alkylendioxy mit vorzugsweise 1 oder 2 Kohlenstoffatomen wie Methylendioxy oder Ethylendioxy; halogensubstituiertes Alkylendioxy mit vorzugsweise 1 oder 2 Kohlenstoffatomen und vorzugsweise 1 bis 4, insbesondere 2 bis 3 Halogenatomen,

wobei die Halogenatome gleich oder verschieden sind und als Halogenatome vorzugsweise Fluor oder Chlor, insbesondere Fluor stehen wie Difluormethylendioxy, Trifluorethylendioxy, Tetrafluorethylendioxy; Hydroxy; Halogen, vorzugsweise Fluor, Chlor, Brom und Jod, insbesondere Chlor und Brom; Cyano; Nitro; Amino; Monoalkyl- und Dialkylamino mit vorzugsweise 1 bis 4, insbesondere 1 oder 2 Kohlenstoffatomen je Alkylgruppe, wie Methylamino, Methyl- ethyl-amino, n.- und i.-Propylamino und Methyl-n.- Butylamino; Formyl; Carboxyl; Alkylcarbonyl mit vorzugsweise 2-4 Kohlenstoffatomen; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Napthylether, die ihrerseits wieder substituiert sein können.

$R^2$ für Wasserstoff, $C_1$-$C_4$-Alkyl, Phenyl, Benzyl, die gegebenenfalls substituiert sein können, steht,

$R^3$ für Wasserstoff, $C_1$-$C_4$-Alkyl, Phenyl, Benzyl, die gegegbenenfalls substituiert sein können, steht,

$R^5$ für Wasserstoff oder $C_1$-$C_4$-Alkyl steht,

$R^6$ für den Fall, daß $X^1$ für O steht, für Phenyl steht, das mindestens einen Substituenten aus der Gruppe

$NO_2$, $C_1$-$C_4$-Halogenalkyl, $C_1$-$C_4$-Alkoxy, Phenyloxy, das gegebenenfalls durch $C_1$-$C_4$-Alkyl, $C_1$-$C_4$-Halogenalkyl, Halogen, $C_1$-$C_4$-Alkoxy, $C_1$-$C_4$-Halogenalkoxy, $C_1$-$C_4$-Halogenalkylthio substituiert ist, $C_1$-$C_4$-Alkylthio, Phenylthio, das gegebenenfalls durch $C_1$-$C_4$-Alkyl, $C_1$-$C_4$- Halogenalkyl, Halogen, $C_1$-$C_4$-Alkoxy, $C_1$-$C_4$-Halogenalkoxy, $C_1$-$C_4$-Halogenalkylthio substituiert ist, $C_1$-$C_4$- Halogenalkoxy, $C_1$-$C_4$-Halogenalkylthio, Methylendioxy, Ethylendioxy, die gegebenenfalls durch Fluor oder Chlor substituiert ist, $C_1$-$C_4$-Alkylsulfenyl, $C_1$-$C_4$-Alkylsulfonyl, $C_1$-$C_4$-Halogenalkylsulfenyl, $C_1$-$C_4$-Halogenalkylsulfonyl, Chlor-, Fluor-, Bromsulfonyl, insbesondere Fluorsulfonyl, und gegebenenfalls noch weitere Substituenten aus der Gruppe Halogen, insbesondere Fluor, Chlor, Brom, $C_1$-$C_4$-Alkyl trägt, unter der Voraussetzung, daß wenn $R^6$ für einen Halogenalkylrest steht, noch weitere Substituenten vorhanden sind.

$R^6$ für den Fall, daß $X^2$ für S steht, für Benzyl steht, das gegebenenfalls einen oder mehrere der folgenden Substituenten trägt:
Alkyl mit vorzugsweise 1 bis 4, insbesondere 1 oder 2 Kohlenstoffatomen, wie Methyl, Ethyl, n.- und i.-Propyl und n.-, i.- und t.-Butyl; Alkoxy mit vorzugsweise 1 bis 4, insbesondere 1 oder 2 Kohlenstoffatomen, wie Methoxy, Ethoxy, n.- und i.-Propyloxy und n.-, i.- und t.-Butyloxy; Alkylthio mit vorzugsweise 1 bis 4, insbesondere 1 oder 2 Kohlenstoffatomen, wie Methylthio, Ethylthio, n.- und i.-Propylthio und n.-, i.- und t.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl, Fluor-, Chlorethyl; Halogenalkoxy mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen wie Trifluormethoxy; Halogenalkylthio mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome vorzugsweise Fluor, Chlor, Brom, insbesondere Fluor stehen, wie Trifluormethylthio; Alkylendioxy mit vorzugsweise 1 oder 2 Kohlenstoffatomen wie Methylendioxy oder Ethylendioxy; halogensubstituiertes Alkylendioxy mit vorzugsweise 1 oder 2 Kohlenstoffatomen und vorzugsweise 1 bis 4, insbesondere 2 bis 3 Halogenatomen, wobei die Halogenatome gleich oder verschieden sind und als Halogenatome vorzugsweise Fluor oder Chlor, insbesondere Fluor stehen wie Difluormethylendioxy, Trifluorethylendioxy, Tetrafluorethylendioxy; Hydroxy; Halogen, vorzugsweise Fluor, Chlor, Brom und Jod, insbesondere Chlor und Brom; Cyano; Nitro;

Amino; Monoalkyl- und Dialkylamino mit vorzugsweise 1 bis 4, insbesondere 1 oder 2 Kohlenstoffatomen je Alkylgruppe, wie Methylamino, Methyl- ethyl-amino, n.- und i.-Propylamino und Methyl-n.- Butylamino; Formyl; Carboxyl; Carbalkoxy mit vorzugsweise 2 bis 4, insbesondere 2 oder 3 Kohlenstoffatomen, wie Carbomethoxy und Carboethoxy; Sulfo ($-SO_3H$); Alkylsulfonyl mit vorzugsweise 1 bis 4, insbesondere 1 oder 2 Kohlenstoffatomen, wie Methylsulfonyl und Ethylsulfonyl; Arylsulfonyl mit vorzugsweise 6 oder 10 Arylkohlenstoffatomen, wie Phenylsulfonyl; Phenyl, Naphthyl, Phenoxy, Naphthoxy, Phenylthio, Napthylether, die ihrerseits wieder substituiert sein können.

Besonders bevorzugt sind Verbindungen der Formel I, in welcher

$X^1$ für O steht,

$R^1$ für Phenoxy steht, das gegebenenfalls einen oder mehrere der folgenden Substituenten trägt: Halogen, insbesondere Fluor, Chlor, Brom, $NO_2$, $C_1$-$C_4$-Alkoxy, insbesondere Methoxy, Ethoxy, $C_1$-$C_4$-Alkyl, insbesondere Methyl, Ethyl, Propyl, $C_1$-$C_4$-Alkylthio, insbesondere Methylthio, Ethylthio, $C_1$-$C_4$- Halogenalkyl, insbesondere Trifluormethyl, $C_1$-$C_4$-Halogenalkoxy, insbesondere Trifluormethoxy, $C_1$-$C_4$-Halogenalkylthio, insbesondere Trifluormethylthio. Bevorzugt stehen dabei die Substituenten in 3- und 4-Stellung des Phenylringes.

$R^2$ für $C_1$-$C_4$-Alkyl, Phenyl, Benzyl steht,

$R^3$ für $C_1$-$C_4$-Alkyl, Phenyl, Benzyl steht,

$X^2$ für O steht,

$R^5$ für Wasserstoff steht,

$R^6$ für Phenyl steht, das mindestens einen Substituenten aus der Gruppe $NO_2$, $C_1$-$C_4$-Halogenalkyl, insbesondere Trifluormethyl, $C_1$-$C_4$-Alkoxy, insbesondere Methoxy, Ethoxy, Phenyloxy, das gegebenenfalls durch $C_1$-$C_4$-Halogenalkyl, insbesondere Trifluormethyl substituiert ist, $C_1$-$C_4$-Alkylthio, insbesondere Methylthio, Ethylthio, $C_1$-$C_4$-Halogenalkoxy, insbesondere Trifluormethoxy, Tetrafluorethoxy, Trichlormethoxy, Fluorchlorethoxy, $C_1$-$C_4$-Halogenalkylthio, insbesondere Trifluormethylmercapto, Methylendioxy oder Ethylendioxy, die gegebenenfalls durch Fluor oder Chlor, insbesondere durch Fluor substituiert sind, $C_1$-$C_4$-Alkylsulfonyl, insbesondere Trifluormethylsulfonyl, Fluorsulfonyl, Chlorsulfonyl, sowie gegebenenfalls weitere Substituenten aus der Gruppe $C_1$-$C_4$-Alkyl, insbesondere Methyl oder Ethyl sowie Halogen, insbesondere Fluor oder Chlor trägt, unter der Voraussetzung, daß wenn $R^6$ für einen Halogenalkylrest steht, noch weitere Substituenten vorhande sind.

Ganz besonders bevorzugt sind Verbindungen der Formel I, in welcher

$X^1$ für O steht,

$R^1$ für Phenoxy steht, das gegebenenfalls in 3- oder 4-Stellung substituiert ist durch Halogen, insbesondere Chlor, $NO_2$, $C_1$-$C_4$-Alkyl, insbesondere Methyl, $C_1$-$C_4$-Alkoxy, insbesondere Methoxy,

$R^2$ für $C_1$-$C_4$-Alkyl, insbesondere Methyl oder Ethyl, Phenyl steht,

$R^3$ für $C_1$-$C_4$-Alkyl, insbesondere Methyl, Ethyl, Propyl, Benzyl steht,

$X^2$ für O steht,

$R^5$ für Wasserstoff steht,

$R^6$ für Phenyl steht, das mindestens einen Substituenten aus der Gruppe $NO_2$, $CF_3$, $OCF_3$, $SCF_3$, $SOCF_3$, $SO_2CF_3$, $SO_2F$, $OCH_3$, $OCF_2CF_2H$, Phenoxy, das durch Trifluormethyl substituiert ist, Fluor-Chlor-substituiertes Ethylendioxy sowie gegebenenfalls weitere Substituenten aus der Gruppe Chlor, Methyl trägt, unter der Voraussetzung, daß wenn $R^6$ für einen Trifluormethylrest steht, noch weitere Substituenten vorhanden sind.

Besonders bevorzugt sind Verbindungen der Formel I, in welcher

$X^1$ für S steht,

$R^1$ für Phenoxy steht, das gegebenenfalls einen oder mehrere der folgenden Substituenten trägt: Halogen, insbesondere Fluor, Chlor, Brom, $NO_2$, $C_{1-4}$-Alkoxy, insbesondere Methoxy, Ethoxy, $C_1$-$C_4$-Alkyl, insbesondere Methyl, Ethyl, Propyl, $C_1$-$C_4$-Alkylthio, insbesondere Methylthio, Ethylthio, $C_1$-$C_4$- Halogenalkyl, insbesondere Trifluormethyl, $C_1$-$C_4$-Halogenalkoxy, insbesondere Trifluormethoxy, $C_1$-$C_4$-Halogenalkylthio, insbesondere Trifluormethylthio. Bevorzugt stehen dabei die Substituenten in 3- und 4-Stellung des Phenylringes.

$R^2$ für $C_{1-4}$-Alkyl, Phenyl, Benzyl steht,

$R^3$ für $C_{1-4}$-Alkyl, Phenyl, Benzyl steht,

$X^2$ für O steht,

$R^5$ für Wasserstoff steht,

$R^6$ für Phenyl steht, das gegebenenfalls substituiert ist durch $C_1$-$C_4$-Alkyl, insbesondere Methyl, $C_1$-$C_4$-Alkoxy, insbesondere Methoxy, Ethoxy, $C_1$-$C_4$-Halogenalkoxy, insbesondere Trifluormethoxy, Fluor-Chlorethoxy, $C_1$-$C_4$-Halogenalkylthio, insbesondere Trifluormethylthio, $C_1$-$C_4$-Alkylthio, insbesondere Methylthio, Halogensulfonyl, insbesondere Fluorsulfonyl, Chlorsulfonyl, $C_1$-$C_4$-Alkylsulfonyl, insbesondere Methylsulfonyl, $C_1$-$C_4$-Halogenalkylsulfonyl, insbesondere Trifluormethylsulfonyl, $C_1$-$C_4$-Halogenalkyl, insbesondere Trifluormethyl, Methylendioxy oder Ethylendioxy,

die gegebenenfalls durch Fluor oder Chlor substituiert sind, Halogen, insbesondere Fluor oder Chlor.

Ganz besonders bevorzugt sind Verbindungen der Formel I, in welcher

$X^1$ für S steht,

$R^1$ für Phenoxy steht, das gegebenenfalls in 3- oder 4-Stellung substituiert ist durch Halogen, insbesondere Chlor, $NO_2$, $C_1$-$C_4$-Alkyl, insbesondere Methyl, $C_1$-$C_4$-Alkoxy, insbesondere Methoxy, $C_1$-$C_4$-Halogenalkyl,

$R^2$ für $C_1$-$C_4$-Alkyl, insbesondere Methyl oder Ethyl, Phenyl steht,

$R^3$ für $C_1$-$C_4$-Alkyl, insbesondere Methyl, Ethyl, Propyl, Benzyl steht,

$X^2$ für O steht,

$R^5$ für Wasserstoff steht,

$R^6$ für Phenyl steht, das gegebenenfalls substituiert ist durch Halogen, insbesondere Chlor, $NO_2$, $CF_3$, $OCF_3$, $SO_2F$, $SCF_3$, $SOCF_3$, $SO_2CF_3$, $OCH_3$, $OCF_2CF_2H$, Phenoxy, das durch Trifluormethyl substituiert ist, Fluor-Chlor-substituiertes Ethylendioxy, Methyl, Ethyl.

Im einzelnen seien folgende Verbindungen der Formel I genannt:

$R^1 =$ 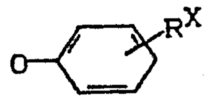

| | | | | | $CX^2NR^5R^6$ | |
|---|---|---|---|---|---|---|
| $X^1$ | $R^X$ | $R^2$ | $R^3$ | $X^2$ | $R^5$ | $R^6$ |
| O | 4-Cl | $-C_6H_5$ | $-CH_3$ | O | H | 3-Cl,4-$CF_3$-$C_6H_3$ |
| O | 4-$CF_3$ | $-CH_3$ | $-C_2H_5$ | O | H | 4-$OCF_2CF_2H$-$C_6H_4$ |
| O | 3-Cl,4-$CF_3$ | $-CH_3$ | $iC_3H_7$ | O | H | 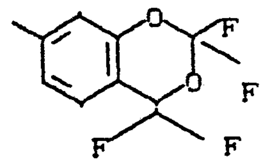 |
| O | 3-Cl | $-CH_3$ | $-CH_3$ | S | $-CH_3$ | 4-$OCF_3C_6H_4$ |
| S | 4-Cl | $-C_2H_5$ | $-C_2H_5$ | O | H | 4-$SCF_3C_6H_4$ |
| S | 4-$CF_3$ | $-CH_3$ | $-CH_3$ | O | H | 3-Cl, 4-$SCF_3$-$C_6H_3$ |
| O | 4-$NO_2$ | $-C_6H_5$ | $-CH_3$ | S | $C_2H_5$ | 3,4-$Cl_2C_6H_3$ |
| O | 4-$CH_3$ | $-CH_3$ | $-CH_3$ | S | H | 4-$OCF_3C_6H_4$ |
| O | 4-$OCF_3$ | $-CH_3$ | $-C_2H_5$ | O | H | 4-$SCF_3C_6H_4$ |
| O | 3,4-$Cl_2$ | $-C_6H_5$ | $-CH_3$ | O | $CH_3$ | 3-Cl, 4-$CF_3C_6H_3$ |
| O | 3,4-$Cl_2$ | $-CH_3$ | $-CH_3$ | O | H | 4-$SCF_3C_6H_4$ |
| O | 3Cl,4$CF_3$ | $-C_6H_5$ | $-C_6H_5$ | O | H | 3-Cl, 4-$SCF_3C_6H_5$ |
| O | 4-$C_6H_5$ | $-CH_3$ | $-CH_3$ | O | H | 4-$SCF_3$-$C_6H_4$ |
| O | 4-(α-$C_{10}H_7$) | $-CH_3$ | $-CH_3$ | O | H | 4-$OCF_3C_6H_4$ |
| O | 4-$CF_3$ | $-4C_5H_4Cl$ | $-CH_3$ | S | H | 4-$OCF_3$-$C_6H_4$ |

Als Basen, mit denen die Verbindungen der Formel I Salze bilden können, seien genannt: Alkali- und Erdalkalihydroxide, Ammoniak, primäre und sekundäre und tertiäre

Picoline, Trimethylamin, Diisopropylamin, Morpholin, Pyrrolidon, Hexamethylenimin.

Setzt man bei Verfahren a) als Diazaphosphorin der Formel II 1,3-Dimethyl-diaza-2,4,6-trioxo-2-(4-chlorphenoxy)-phospha-cyclohexan und als Isocyanat der Formel III 4-Trifluormethoxyphenylisocyanat ein, läßt sich das Verfahren a) durch folgendes Formelschema wiedergeben:

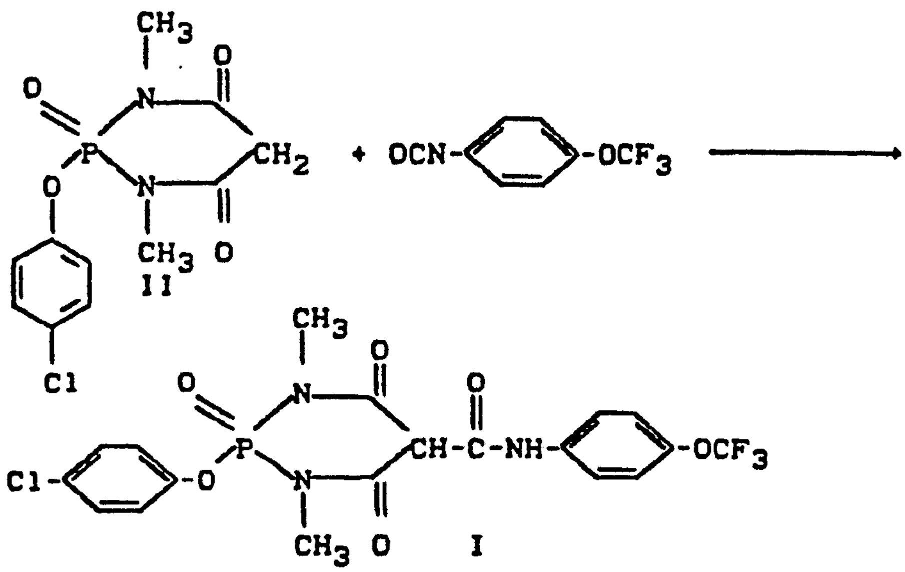

II

I

Diazaphosphorine der Formel II sind bekannt oder lassen sich analog zu bekannten Verfahren herstellen (CH-PS 643 563 und DE-OS 2 600 665). Sie lassen sich herstellen durch Umsetzung von entsprechend substituierten Phosphorsäureesterdiamide mit Malonylhalogenid.

Bevorzugt werden Diazaphosphorine der Formel II eingesetzt, in denen die Substituenten die bei den Verbindungen der Formel I genannten bevorzugten und besonders bevorzugten Bedeutungen besitzen.

Im einzelnen seien folgende Diazaphosphorine der Formel II genannt:

Im einzelnen seien folgende Diazaphosphorine der Formel II genannt:
2-(4-Chlorphenoxy)-2,4,6-trioxo-1,3-dimethyl-1,3,2-diazaphosphorin, 2-(3-Chlorphenoxy)-2,4,6-trioxo-1-methyl-3-isopropyl-1,3,2-diazaphosphorin, 2-(3,4-Dichlorphenoxy)-2,4,6-trioxo-1,3-dimethyl-1,3,2-diazaphosphorin, 2-(4-Trifluormethylphenoxy)-2,4,6-trioxo-1,3-dimethyl-1,3,2-diazaphosphorin, 2-(4-Nitrophenoxy)-2,4,6-trioxo-1,3-dimethyl-1,3,2-diazaphoshorin; 2-(4-Chlorphenoxy)-2,4,6-trioxo-1-phenyl-3-methyl-1,3,2-diazaphosphorin; 2-(3-Tolyloxy)-2,4,6-trioxo-1,3-diisopropyl-1,3,2-diazaphosphorin; 2-(4-Trifluormethoxyphenoxy)-2,4,6-trioxo-1,3-dimethyl-1,3,2-diazaphosphorin; 2-(3-Chlorphenoxy)-2,4,6-trioxo-1-methyl-3-ethyl-1,3,2-diazaphosphorin; 2-(β-Naphtoxy)2,4,6-trioxo-1,3-dimethyl-1,3,2-diazaphosphorin; 2-(4-Phenylphenoxy)-2,4,6-trioxo-1-methyl-3-isopropyl-1,3,2-diazaphosphorin; 2-(4-Chlorphenoxy)-2-thioxo-4,6-dioxo-1,3-dimethyl-1,3,2-diazaphosphorin; 2-(3-Chlorphenoxy)-2-thiooxo-4,6-dioxo-1-methyl-3-isopropyl-1,3,2-diazaphosorin.

Isocyanate der Formel III sind bekannt oder lassen sich analog in bekannten Verfahren herstellen. Bevorzugt werden Isocyanate eingesetzt, in denen die Substituenten $R^6$ die bei den Verbindungen der Formel I genannten bevorzugten und besonders bevorzugten Bedeutungen besitzen.

Im einzelnen seien folgende Isocyanate der Formel III genannt:
4-Nitrophenylisocyanat, 3-Chlor-4-trifluormethyl-isocyanat, 3-Trifluormethyl-4-Chlor-isocyanat, 4-Trifluormethoxyphenylisocyanat, 4-Trifluormercaptomethylphenylisocyanat, 3-Chlor-4-trifluormethoxyphenylisocyanat,

3-Chlor-4-trifluormercaptomethylphenylisocyanat, 3-Nitro-4-trifluormethylphenylisocyanat, 4-Trifluormethylsulfinylphenylisocyanat, Trifluormethyl-4-iso-cyanatophenyl)-sulfon, 4-(1,1,2,2-Tetrafluorethoxy)-phenylisocyanat, 2,6-Dichlor-4-trifluormercaptomethyl-phenylisocyanat; 4-(4-Trifluormethylphenoxy)-phenylisocyanat; 4-(3-Trifluormethylphenoxy)-phenylisocyanat;

Die Verbindungen der Formeln II und III werden vorzugsweise in Gegenwart von Verdünnungsmitteln und von Katalysatoren umgesetzt.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Diemthylformamid, Dimethylacetamid und N-methylpyrrolidon, sowie Dimethylsulfoxid, Tetramethylensulfon und Hexamethylphosphorsäuretriamid.

Als Katalysatoren kommen die bei Umsetzungen mit Isocyanaten üblichen Katalysatoren infrage. Als solche seien genannt: Tert.-Amine wie Triethylamin, N-Methylmorpholin, 1,4-Diaza-bicyclo-(2,2,2)-octan (DABCO) β,β'-Dimethylaminodiethylether, Dimethylbenzylamin, DBN = 1,5-Diazabicyclo(4,3,0)-non-5-en, DBU = 1,8-Diazabicyclo(5,4,0)-undec-7-en,Metallkatalysatoren des Zn, Sn, Pb wie Dibutylzinndilaurat, Dibutylzinndioxid, Zinnoctoat, Bleioctoat, Zinkoctoat, Zinkchlorid, Zinkacetat.

Die Reaktion wird zwischen 50 und 150°C bevorzugt zwischen 60-110°C durchgeführt. Man arbeitet vorzugsweise unter Normaldruck.

Die Verbindungen der Formeln II und III bzw. IV und V werden in äquimolaren Mengen eingesetzt ein geringer Überschuß der einen oder anderen Komponente bringt keine wesentlichen Vorteile.

Die Aufarbeitung erfolgt in an sich bekannter Weise, z.B. durch Versetzen der Reaktionsmischung mit Wasser, Abtrennen der organischen Phase und Abdestillieren des Lösungsmittels.

Setzt man bei Verfahren b) als Diazaphosphorin der Formel IV 1-Methyl-3-isopropyl-diaza-2,4,6-trioxo-2-(4-bromphenoxy)-5-methoxycarbonyl-2-phosphacyclohexan und als Anilin der Formel V 3-Trifluormethyl-4-trifluormethoxyanilin ein, läßt sich das Verfahren b) durch folgendes Formelschema wiedergegeben:

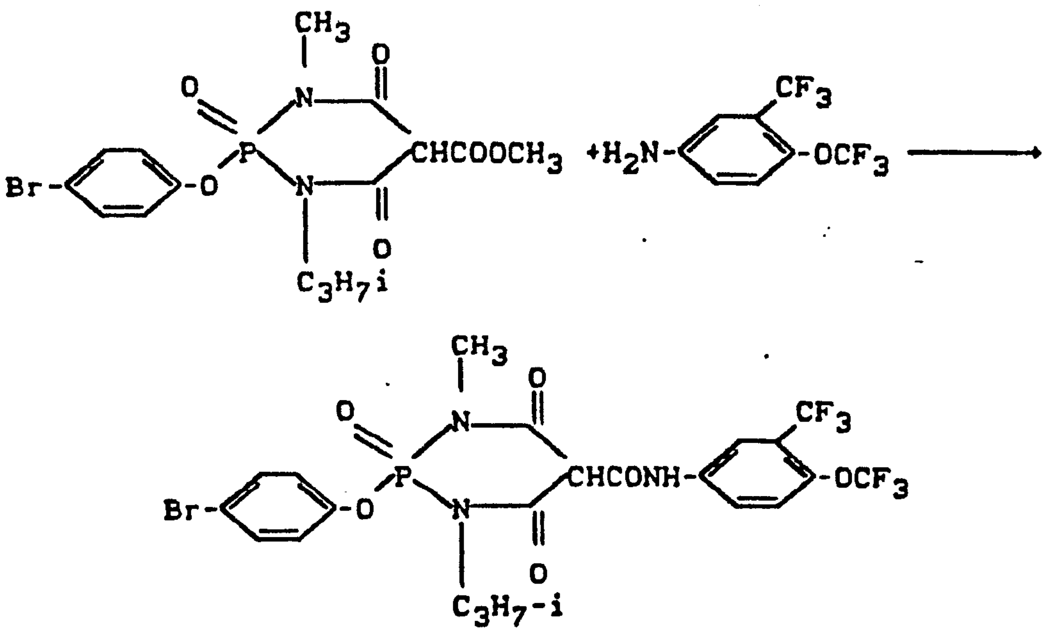

Die Verbindungen der Formel IV sind bekannt oder lassen sich analog zu bekannten Verfahren herstellen (CH-PS 643 563).

Bevorzugt werden Verbindungen der Formel IV eingesetzt, in denen die Substituenten die bei den Verbindungen der Formel I genannten bevorzugten und besonders bevorzugten Bedeutungen besitzen.

Im einzelnen seien folgende Verbindungen der Formel IV genannt:

Die entsprechenden 5-Ethoxycarbonyle der folgenden Verbindungen:

2-(4-Chorphenoxy)-2,4,6-trioxo-1,3-dimethyl-1,3,2-diazaphosphorin; 2-(3-Chlorphenoxy)-2,4,6-trioxo-1-methyl-3-isopropyl-1,3,2-diazaphosphorin, 2-(3,4-Dichlorphenoxy)-2,4,6-trioxo-1,3-dimethyl-1,3,2-diazaphosphorin, 2-(4-Trifluormethylphenoxy)-2,4,6-trioxo-1,3-dimethyl-1,3,2-diazaphosphorin; 2-(4-Nitrophenoxy)-2,4,6-trioxo-1,3-dimethyl-1,3,2-diazaphosphorin; 2-(4-Chlorphenoxy)-2,4,6-trioxo-1-phenyl-3-methyl-1,3,2-diazaphosphorin; 2-(3-Tolyloxy)-2,4,6-trioxo-1,3-diisopropyl-1,3,2-diazaphosphorin; 2-(4-Trifluormethoxyphenoxy)-2,4,6-trioxo-1,3-dimethyl-1,3,2-diazaphosphorin; 2-(3-Chlorphenoxy)-2,4,6-trioxo-1-methyl-3-ethyl-1,3,2-diazaphosphorin; 2-(β-Naphthoxy)-2,4,6-trioxo-1,3-dimethyl-1,3,2-diazaphosphorin; 2-(4-phenylphenoxy)-2,4,6-trioxo-1-methyl-3-isopropyl-1,3,2-diazaphosphorin; 2-(4-Chlorphenoxy)-2-thioxo-4,6-dioxo-1,3-dimethyl--1,3,2-diazaphosphorin; 2-(3-Chlorphenoxy)-2-thiooxo-4,6-dioxo-1-methyl-3-isopropyl-1,3,2-diazaphosphorin.

Die Amine der Formel V sind bekannt oder lassen sich analog zu bekannten Verfahren herstellen.

Bevorzugt werden Amine der Formel V eingesetzt, in denen die Substituenten $R^5$ und $R^6$ die bei den Verbindungen der Formel I genannten bevorzugten und besonders bevorzugten Bedeutungen besitzen.

Im einzelnen seien folgende Verbindungen der Formel V genannt:

4-Nitroanilin, 3-Chlor-4-trifluormethylanilin, 3-Triflurmethyl-4-chloranilin, 4-Trifluormethoxy anilin, 4-Trifluormercaptomethylanilin, 3-Chlor-4-trifluormethoxy anilin, 3-Chlor-4-trifluormercaptomethylanilin, 3-Nitro-4-trifluormethylanilin, 4-Trifluormethylsulfinylanilinisocyanat, Trifluormethyl-4-aminophenyl-sulfon,

4-(1,1,2,2-Tetrafluorethoxy)-anilin, 2,6-Dichlor-4-trifluor mercaptomethyl-anilin, 4-Amino-4'-trifluormethyldiphenylether, 4-Amino-3'-trifluormethyl-diphenylether;

Die Umsetzung der Verbindungen der Formel IV und V erfolgt vorzugsweise in Gegenwart von Verdünnungsmitteln sowie in Gegenwart von Basen.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Chloroform, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl- und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Diemthylformamid, Dimethylacetamid und N-methylpyrrolidon, sowie Dimethylsulfoxid, Tetramethylensulfon und Hexamethylphosphorsäuretriamid, ferner Alkohole wie Methanol, Ethanol, Propanol, Butanol.

Als Basen seien genannt Alkali- und Erdalkalihydroxide, Alkali- und Erdalkalialkoholate.

Die Reaktion wird zwischen 50 und 150°C bevorzugt zwischen 60-110°C durchgeführt. Man arbeitet vorzugsweise unter Normaldruck.

Die Verbindungen der Formeln IV und V werden in äquimolaren Mengen eingesetzt ein geringer Überschuß der einen oder anderen Komponente bringt keine wesentlichen Vorteile.

Die Aufarbeitung erfolgt in an sich bekannter Weise, z.B. durch Versetzen der Reaktionsmischung mit Wasser, Abtrennen der organischen Phase und Abdestillieren des Lösungsmittels.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.

Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.
Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z.B. Diprion spp., Hoplocampa spp., Lasius spp., Monomorium pharaonis, Vespa spp.

Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..

Aus der Ordnung der Arachnida z.B. Scorpio maurus, Latrodectus mactans.

Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die hergestellten Wirkstoffe weisen auch gute fungizide Eigenschaften auf, so z.B. gegen Pyricularia oryzae in Reis und gegen Apfelschorf und Oomyceten.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht in der Veterinärmedizin in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuderns sowie durch parenterale Anwendung in Form beispielsweise der Injektion.

Die erfindungsgemäßen Wirkstoffe sind breit wirksam gegen Endoparasiten. Sie wirken vor allem gegen Trematoden und Nematoden, insbesondere Leberegel und Magen- und Darmnematoden der Wiederkäuer. Darüber hinaus wirken sie auch gegen solche Magen- und Darmnematoden, die gegen die gebräuchlichen Benzimidazol-Anthelmintika resistent und damit nicht mehr ausreichend therapierbar sind.

Die Wirkung wurde im Tierversuch nach oraler, parenteraler und dermaler Applikation bei stark mit Parasiten befallenen Versuchstieren geprüft. Die angewendeten Dosierungen wurden sehr gut von den Versuchstieren vertragen.

Die erfindungsgemäßen Wirkstoffe können als Anthelmintika verwendet werden.

Die erfindungsgemäßen Wirkstoffe können zusammen mit anderen üblichen Anthelmintika verabreicht werden.

Die erfindungsgemäßen Wirkstoffe können entweder als solche oder aber in Kombination mit pharmazeutisch annehmbaren Trägern zur Anwendung gelangen. Als Darreichungsformen in Kombination mit verschiedenen inerten Trägern kommen Tabletten, Kapseln, Granulate, wäßrige Suspensionen, injizierbare Lösungen, Emulsionen und Suspensionen, Elixiere, Sirup, Pasten und dergleichen in Betracht. Derartige Träger umfassen feste Verdünnungsmittel oder Füllstoffe, ein steriles, wäßriges Medium sowie verschiedene nicht toxische organische Lösungsmittel und dergleichen. Selbstverständlich können die für eine orale Verabreichung in Betracht kommenden Tabletten und dergleichen mit Süßstoffzusatz und ähnlichen versehen werden. Die therapeutisch wirksame Verbindung soll im vorgenannten Fall in einer Konzentration von etwa 0,5 bis 90 Gew.-% der Gesamtmischung vorhanden sein, d.h. in Mengen, die ausreichend sind, um den obengenannten Dosierungsspielraum zu erreichen.

Die Formulierungen werden in bekannter Weise hergestellt, z.B. durch Verstrecken der Wirkstoffe mit Lösungsmitteln und/oder Trägerstoffen, gegebenenfalls unter Verwendung

von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielhaft aufgeführt: Wasser, nicht toxische organische Lösungsmittel, wie Paraffine (z.B. Erdölfraktionen), pflanzliche Öle (z.B. Erdnuß-(Sesamöl)), Alkohole (z.B. Ethylalkohol, Glycerin), Glykole (z.B. Propylenglykol, Polyethylenglykol) und Wasser; feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Roh-, Milch- und Traubenzucker); Emulgiermittel, wie nicht ionogene und anionische Emulgatoren (z.B. Polyoxyethylen-Fettsäure-Ester, Polyoxyethylen-Fettalkohol-Ether, Alkylsulfonate und Arylsulfonate), Dispergiermittel (z.B. Methylcellulose, Stärke und Polyvinylpyrrolidon) und Gleitmittel (z.B. Magnesiumstearat, Talkum, Stearinsäure und Natriumlaurylsulfat).

Im Falle der oralen Anwendung können Tabletten selbstverständlich außer den genannten Trägerstoffen auch Zusätze wie Natriumcitrat, Calciumcarbonat und Dicalciumphosphat, zusammen mit verschiedenen Zuschlagstoffen, wie Stärke, vorzugsweise Kartoffelstärke, Gelatine und dergleichen, enthalten. Weiterhin können Gleitmittel, wie Magnesiumstearat, Natriumlaurylsulfat und Talkum, zum Tablettieren mitverwendet werden.

Im Falle wäßriger Suspensionen und/oder Elixieren, die für die orale Anwendung gedacht sind, können die Wirkstoffe außer mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Die Wirkstoffe können in Kapseln, Tabletten, Pastillen, Dragees, Ampullen usw. auch in Form von Dosierungseinheiten enthalten sein, wobei jede Dosierungseinheit so angepaßt ist, daß sie eine einzelne Dosis des aktiven Bestandteils liefert.

Die erfindungsgemäßen Wirkstoffe können in den Formulierungen auch in Mischungen mit anderen in der Veterinär- und/oder Humanmedizin zur Behandlung von Infektionen und/oder Erkrankungen benutzten bekannten Wirkstoffen vorliegen, insbesondere L-2,3,5,6-Tetrahydro-6-phenylimidazolthiazol, Benzimidazolcarbamaten, Praziquantel, Febantel.

Die Wirkstoffe können in üblicher Weise angewendet werden. Die Applikation erfolgt vorzugsweise oral, eine parenterale, insbesondere subkutane, aber auch eine dermale Applikation (pour-on, spot-on) sind jedoch ebenfalls möglich.

Im allgemeinen hat es sich als vorteilhaft erwiesen, Mengen von etwa 1 bis etwa 100 mg der Wirkstoffe je kg Körpergewicht pro Tag zur Erzielung wirksamer Ergebnisse zu verabreichen.

Trotzdem kann es gegebenenfalls erforderlich sein, von den genannten Mengen abzuweichen, und zwar in Abhängigkeit vom Körpergewicht des Versuchstieres bzw. der Art des Applikationswweges, aber auch aufgrund der Tierart und deren individuellem Verhalten gegenüber dem Medikament bzw. der Art von dessen Formulierung und dem Zeitpunkt bzw. Intervall, zu welchem die Verarbreichung erfolgt. So kann es in einigen Fällen ausreichend sein, mit weniger als der vorgenannten Mindestmenge auszukommen, während in anderen Fällen die genannte obere Grenze überschritten werden muß. Im Fall der Applikation größerer Mengen kann es empfehlenswert sein, diese in mehreren Einzelgaben über den Tag zu verteilen. Für die Applikation in der Veterinärmedizin ist der gleiche Dosierungsspielraum vorgesehen. Sinngemäß gelten auch die weiteren obigen Ausführungen.

0216105

Beispiel A

Phaedon-Larven-Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlpflanzen (Brassica oleracea) werden mit der Wirkstoffzubereitung der gewünschten Konzentration behandelt. Ein Blatt der behandelten Pflanze wird in eine Plastikdose gelegt und mit Larven ($L_3$) des Meerrettichkäfers (Phaedon cochleariae) besetzt. Nach 2 und 4 Tagen wird jeweils ein weiters Blatt von derselben Pflanze für die Nachfütterung verwendet.

Nach der gewünschten Zeit wird die Abtötung in % bestimmt. Dabei bedeutet 100 %, daß alle Käferlarven abgetötet wurden.

Bei diesem Test zeigen z.B. die folgenden Verbindungen der Herstellungsbeispiele überlegene Wirksamkeit gegenüber dem Stand der Technik:

8, 10, 12, 15, 16, 24, 25 und 43.

0216105

Beispiel B

Spodoptera - Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentrationen.

Kunstfutterscheiben, die in Plastikdosen liegen, werden durch Beträufeln mit der Wirkstoffzubereitung der gewünschten Konzentration behandelt. Nach Eindringen der Lösung besetzt man die Plastikdosen mit je 1 Raupe ($L_5$) der Art Spodoptera frugiperda, wobei je Versuchsglied 5 Wiederholungen aufgestellt werden. Nach der Verpuppung legt man die Tiere eines Versuchsglieds in eine größere Dose. Nach der gewünschten Zeit wird die Abtötung in % bestimmt. Dabei bedeutet 100 %, daß alle Tiere abgetötet wurden; 0 % bedeutet, daß keine Tiere abgetötet wurden.

Bei diesem Test zeigen z.B. die folgenden Verbindungen der Herstellungsbeispiele überlegene Wirksamkeit gegenüber dem Stand der Technik:

7, 9, 10, 16, 17, 24, 27, 28, 29, 30, 32, 34, 35 und 36.

## Beispiel C

Aedes - Test

Lösungsmittel: 3 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentrationen.

Man füllt die wässrigen Wirkstoffzubereitungen in Gläser und setzt anschließend 20 Mückenlarven ($L_4$) (Aedes aegypti) in jedes Glas ein.

Nach der gewünschten Zeit wird die Abtötung in % bestimmt. Dabei bedeutet 100 %, daß alle Tiere abgetötet wurden; 0 % bedeutet, daß keine Tiere abgetötet wurden.

Bei diesem Test zeigen z.B. die folgenden Verbindungen der Herstellungsbeispiele überlegene Wirksamkeit gegenüber dem Stand der Technik:

6, 7, 8, 9, 10, 12, 16, 25 und 26.

Beispiel D

Fliegenmaden-Entwicklungshemmungstest

Testtiere: Musca domestica-(multiresistent) Maden

Lösungsmittel: Aceton

2 Gewichtsteile Wirkstoff werden in 1 000 Volumenteilen Lösungsmittel aufgenommen. Die so erhaltene Lösung wird mit weiteren Lösungsmitteln auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man etwa 25 Testtiere in die Petrischale und bedeckt sie mit einem Glasdeckel.

14 Tage nach Ansetzen der Versuche wird die Zahl der geschlüpften Fliegen kontrolliert. Bestimmt wird der %-Satz der Schlüpfhemmung im Vergleich zur unbehandelten Kontrolle.

In diesem Test zeigen z.B. die folgenden Verbindungen der Herstellungsbeispiele überlegene Wirksamkeit gegenüber dem Stand der Technik:

6,7, 24, 25, 26, 28 und 30.

0216105

Beispiel F

Test mit Lucilia cuprina resistent-Larven

Emulgator: 35 Gewichtsteile Ethylenglykolmonomethylether
35 Gewichtsteile Nonylphenolpolyglykolether

Zur Herstellung einer zweckmäßigen Wirkstoffzubereitung vermischt man drei Gewichtsteile Wirkstoff mit sieben Gewichtsteilen des oben angegebenen Gemisches und verdünnt das so erhaltene Konzentrat mit Wasser auf die jeweils gewünschte Konzentration.

Etwa 20 Lucilia cuprina res.-Larven werden in ein Teströhrchen gebracht, welches ca. 1 $cm^3$ Pferdefleisch und 0,5 ml der Wirkstoffzubereitung enthält. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 8, 24, 25, 26, 33, 34, 41.

Beispiel F

In vitro Nematodentest

Caenorhabditis elegans

$10^{-4}$ g Wirkstoff wurden in 1 ml Wasser oder 0,1 ml Dimethylsulfoxid (DMSO) gelöst. Diese Lösung wurde auf eine Replica-Platte gegeben. Dazu wurden 2 ml einer E-coli-Suspension gegeben, in der man 10-20 weibliche Tiere oder Larven von Caenorhabditis elegans in 0,5 ml M9 Pufferlösung gegeben hat. Die E-coli-Suspension wurde hergestellt indem man 300 ml einer Übernachthalter eines Uracil-bedürftigen E-coli-Stammes mit 1,8 l steriler M 9 Pufferlösung versetzte.

Der Versuchsansatz wurde 7 Tage bei 22°C inkubiert und danach ausgewertet. Es wurde bewertet inwieweit der Wirkstoff die Vermehrung beeinträchtigt und die Konzentration angegeben bei der die Vermehrung verhindert wird. Dabei wurden folgende Ergebnisse erhalten:

0216105

Tabelle F

In vitro-Nematodentest
Caenorhabditis elegans

| Wirkstoff Bsp. Nr. | Dosis effectiva mg/l |
|---|---|
| 4 | 10 |
| 5 | 10 |
| 6 | 10 |
| 7 | 10 |
| 8 | 10 |
| 9 | 10 |
| 10 | 10 |
| 11 | 100 |
| 12 | 10 |
| 13 | 10 |
| 15 | 10 |
| 16 | 10 |
| 17 | 10 |
| 18 | 100 |
| 19 | 100 |
| 20 | 10 |
| 24 | 10 |
| 26 | 100 |
| 30 | 10 |
| 33 | 10 |
| 34 | 100 |
| 37 | 10 |
| 38 | 10 |

0216105

Tabelle F

In vitro-Nematodentest
Caenorhabditis elegans

| Wirkstoff Bsp. Nr. | Dosis effectiva mg/l |
|---|---|
| 41 | 10 |
| 42 | 10 |
| 44 | 10 |
| 46 | 10 |
| 51 | 100 |
| 54 | 10 |
| 55 | 10 |
| 56 | 10 |
| 57 | 10 |
| 58 | 10 |
| 59 | 10 |
| 61 | 10 |
| 62 | 100 |
| 63 | 10 |
| 64 | 10 |
| 65 | 10 |

0216105

Beispiel G

In vivo Nematodentest

Strongyloides ratti

Experimentell mit Strongyloides ratti infizierte Ratten werden 7 Tage nach der Infektion an drei aufeinanderfolgenden Tagen oral mittels Schlundsonde behandelt. Die Tiere werden 13 Tage nach der Infektion getötet und die Zahl der Parasiten bestimmt. Es wird die Nährstoffkonzentration angegeben, bei der 95% der Parasiten abgetötet wurden (effektive Dosis):

| Wirkstoff Beispiel Nr. | effektive Dosis mg/kg |
|---|---|
| 7 | 50 |
| 9 | 25 |
| 12 | 100 |
| 24 | 10 |
| 25 | 25 |

0216105

Beispiel H

In vivo Nematodentest

Hymenolepis nana

Experimentell mit Hymenolepis nana infizierte Mäuse werden 10 Tage nach der Infektion an vier aufeinanderfolgenden Tagen oral mittels Schlundsonde behandelt. Die Tiere werden 17 Tage nach Infektion getötet und die Zahl der Parasiten bestimmt. Es wird die Wirkstoffkonzentration angegeben, bei der 95% der Parasiten abgetötet wurden (effektive Dosis):

| Wirkstoff Beispiel Nr. | effektive Dosis mg/kg |
|---|---|
| 9 | 50 |

0216105

Beispiel I

In vivo Nematodentest

Heterakis spumosa

Experimentell mit Heterakis spumosa infizierte Mäuse werden 32 Tage nach der Infektion an vier aufeinanderfolgenden Tage oral mittels Schlundsonde behandelt. Die Tiere werden 39 Tage nach der Infektion getötet und die Zahl der Parasiten bestimmt. Es wird die Wirkstoffkonzentration angegeben bei der 95% der Parasiten abgetötet wurden (effektive Dosis):

| Wirkstoff Beispiel Nr. | effektive Dosis mg/kg |
|---|---|
| 9 | 25 |
| 12 | 25 |

0216105

Beispiel K

In vivo/Nematodentest

Haemonchus contortus / Schaf

Experimentell mit normal empfindlichen bzw. mit gegen Benzimidazol-Anthelmintika resistenten Haemonchus contortus infizierte Schafe wurden nach Ablauf der Präpatenzzeit des Parasiten behandelt. Die Wirkstoffe wurden als reiner Wirkstoff in Gelatinkapseln oral appliziert.

Der Wirkungsgrad wird dadurch bestimmt, daß man die mit dem Kot ausgeschiedenen Wurmeier vor und nach der Behandlung quantitativ auszählt.

Ein völliges Sistieren der Eiausscheidung nach der Behandlung bedeutet, daß die Würmer abgetrieben wurden oder so geschädigt sind, daß sie keine Eier mehr produzieren.

| Wirkstoff Beispiel Nr. | Dosis effectiva (mg/kg) |
|---|---|
| 7 | 50 |
| 9 | 25 |
| 12 | 100 |
| 24 | 10 |
| 25 | 25 |
| 12 | 25 |
| 3 | 50 |
| 47 | 10 |

**Beispiele:**

a) Allgemeine Vorschrift zur Herstellung der erfindungsgemäßen 5-Carbamoyl-substituierten 1,3,2-Diazaphosphorine gemäß Verfahren a)

Je 0,03 Mol des 1,3,2-Diazaphosphorins und des Isocyanats werden in 120 ml trockenem THF gelöst vorgelegt und bei Raumtemperatur innerhalb von 30 min. mit einer Lösung von 0,03 Mol (4,56 g) "DBU" in 30 ml THF versetzt. Die anfänglich hellbraune Lösung verdunkelt sich und es tritt eine leichte Wärmeentwicklung auf, so daß die Innentemperatur durch Wasserkühlung unter 25°C gehalten werden muß. Anschließend wird bei Raumtemperatur bis zum vollständigen Umsatz nachgerührt (ca. 4-5 h, DC-Probe!). Dann wird der gesamt Ansatz eingedampft, in 100 ml Methylenchlorid aufgenommen und mit 100 ml verdünnter Salzsäure (10 %ig verrührt. Die organische Phase wird abgetrennt, mit wäßriger Natriumhydrogencarbonatlösung neutral gewaschen und über Natriumsulfat getrocknet. Nach dem Entfernen des Lösungsmittels am Rotationsverdampfer wird der Rückstand aus Ligroin oder Isopropanol umkristallisiert oder chromatographiert.

b) Allgemeine Vorschrift zur Herstellung der erfindungsgemäßen 5-Carbamoyl-substituierten 1,3,2-Diazaphosphorine gemäß Verfahren b)

0,05 Mol des 5-Ethoxycarbonyl-1,3,2-diazaphosphorins (IV) und 0,05 Mol des Anilins (V) werden in 100 ml Toluol suspendiert und unter Inertgas (Stickstoff,

Argon) 10 Stunden am Rückfluß erhitzt, wobei Ethanol abdestilliert wird.

Dann wird das Reaktionsgemisch auf Raumtemperatur abgekühlt und das Lösungsmittel abdestilliert. Der Rückstand wird aus Ligroin/Ethanol umkristallisiert oder chromatographiert.

Gemäß einem der obengenannten Verfahren werden die folgenden Verbindungen hergestellt:

0216105

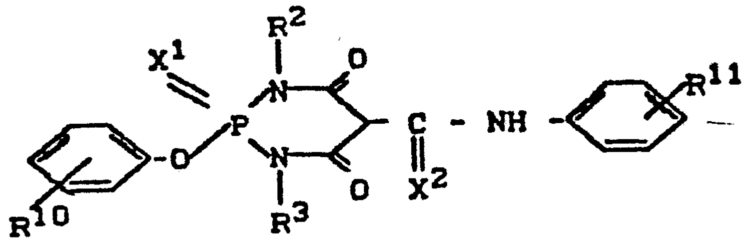

| Beispiel Nr. | $X^1$ | $R^{10}$ | $R^2$=$R^3$ | $X^2$ | $R^{11}$ | Fp/°C |
|---|---|---|---|---|---|---|
| 1 | O | 3,4-$Cl_2$ | $CH_3$ | O | 4-$SCF_3$ | 126 |
| 2 | O | 4-Cl | $CH_3$ | O | 4-($OC_6H_4$-3$CF_3$) | 185 |
| 3 | O | 4-Cl | $CH_3$ | O | 4-($OC_6H_4$-4$CF_3$) | 122 |
| 4 | O | 4-Cl | $CH_3$ | O | 4-$NO_2$ | 145 |
| 5 | O | 4-Cl | $CH_3$ | O | 4-$SO_2CF_3$ | 155 |
| 6 | S | 4-Cl | $CH_3$ | O | 4-Cl,3-$CF_3$ | 145 |
| 7 | S | H | $CH_3$ | O | 4-$OCF_3$ | 111 |
| 8 | O | 4-Cl | $CH_3$ | O | 4-$SCF_3$ | 145-146 |
| 9 | O | 4-Cl | $CH_3$ | O | 3-$CF_3$,4-Cl | 140-142 |
| 10 | O | H | $CH_3$ | O | 4-$SCF_3$ | 118-120 |
| 11 | O | H | $CH_3$ | O | 2-$SCF_3$ | 85-87 |
| 12 | O | H | $CH_3$ | O | 3-$CF_3$,4-Cl | 129-131 |
| 13 | O | H | $CH_3$ | O | 4-$SO_2CF_3$ | 153 |
| 14 | O | H | $CH_3$ | O | 4-$SO_2F$ | 173-175 |
| 15 | O | H | $CH_3$ | O | 4-$OCF_2CF_2H$ | 110-112 |
| 16 | O | H | $CH_3$ | O | 4-$OCF_3$ | 96 |
| 17 | O | 4-Cl | $CH_3$ | O | 4-$OCF_3$ | 112 |
| 18 | O | 4-$NO_2$ | $CH_3$ | O | 4-$NO_2$ | 120 |
| 19 | O | 4-$OCH_3$ | $CH_3$ | O | 4-$OCH_3$ | 116 |
| 20 | O | 4-$CH_3$ | $CH_3$ | O | 4-$OCH_3$ | 117 |

| Beispiel Nr. | $X^1$ | $R^{10}$ | $R^2=R^3$ | $X^2$ | $R^{11}$ | Fp/°C |
|---|---|---|---|---|---|---|
| 21 | O | 4-$NO_2$ | $CH_3$ | O | 4-$OCH_3$ | 147 |
| 22 | O | 4-$OCH_3$ | $CH_3$ | O | 4-$NO_2$ | 191 |
| 23 | O | 4-$CH_3$ | $CH_3$ | O | 4-$NO_2$ | 134 |
| 24 | O | 4-Cl | $CH_3$ | O | 4-$SCF_3$ | * |
| 25 | O | 4-Cl | $CH_3$ | O | 3-$CF_3$,4-Cl | * |
| 26 | O | 4-Cl | $CH_3$ | O | 4-$SO_2CF_3$ | * |
| 27 | O | 4-$CH_3$ | $CH_3$ | O | 3-$CF_3$,4-Cl | 127 |
| 28 | S | H | $CH_3$ | O | 4-$SCF_3$ | 94 |
| 29 | S | H | $CH_3$ | O | 3-$CF_3$,4-Cl | 94 |
| 30 | S | 4-Cl | $CH_3$ | O | 4-$OCF_3$ | 102 |
| 31 | O | 4-$CH_3$ | $CH_3$ | O | 4-$OCH_3$ | 160* |
| 32 | S | 4-Cl | $CH_3$ | O | 4-$SCF_3$ | 87 |
| 33 | S | H | $CH_3$ | O | 4-$SCF_3$ | 158* |
| 34 | S | 4-Cl | $CH_3$ | O | 4-$SCF_3$ | 159* |
| 35 | S | H | $CH_3$ | O | 3-$CH_3$, 4-O-$C_6H_5$-$SCF_3$ | 99 |
| 36 | O | 4-Cl | $CH_3$ | O | 3,4-O-$CF_2$CFCl-O- | 108 |
| 37 | O | 3-Cl | $CH_3$ | O | 4-$SCF_3$ | 101-103 |
| 38 | O | 3-Cl | $CH_3$ | O | 4-$OCF_3$ | 98-100 |
| 39 | O | 4-Cl | $C_6H_5$ | O | 4-$SCF_3$ | 165-166 |
| 40 | O | H | $C_6H_5$ | O | 4-$SCF_3$ | 177-176 |

* Die Verbindungen 24, 25, 26, 31, 33, 34 liegen in Form ihrer Salze mit $H_2NCH(CH_3)_2$ vor.

| Beispiel Nr. | $X^1$ | $R^{10}$ | $R^2$ | $R^3$ | $X^2$ | $R^{11}$ | Fp/°C |
|---|---|---|---|---|---|---|---|
| 41 | O | 3-Cl | i-$C_3H_7$ | $CH_3$ | O | 3-Cl,4-$CF_3$ | Öl |
| 42 | O | 3-Cl | i-$C_3H_7$ | $CH_3$ | O | 4-$SCF_3$ | 80-83 |
| 43 | O | 3-Cl | i-$C_3H_7$ | $CH_3$ | O | 4-$OCF_3$ | Öl |
| 44 | O | H | $CH_3$ | $C_6H_5$ | O | 4-$SCF_3$ | 142-143 |
| 45 | O | 3-Cl | $CH_3$ | i-$C_3H_7$ | O | 3-Cl,4-$SCF_3$ | Öl |
| 46 | O | 3-Cl | $CH_3$ | $C_2H_5$ | O | 4(4'-$OC_6H_4CF_3$) | Öl |
| 47 | O | 3-Cl | $CH_3$ | $C_2H_5$ | O | 4(3'-$OC_6H_4CF_3$) | Öl |
| 48 | S | 3-$CH_3$ | $CH_3$ | i-$C_3H_7$ | O | 4(4'-$OC_6H_4CF_3$) | Öl |
| 49 | S | 3-$CH_3$ | i-$C_3H_7$ | $CH_2C_6H_5$ | O | 3-$CF_3$,4-Cl | Öl |
| 50 | O | 3-Cl | i-$C_3H_7$ | $CH_2C_6H_5$ | O | 4(4'-$OC_6H_4CF_3$) | Öl |
| 51 | O | 3-Cl | i-$C_3H_7$ | $CH_2C_6H_5$ | O | 2,4$(CF_3)_2$ | Öl |
| 52 | O | 3-Cl | i-$C_3H_7$ | $CH_2C_6H_5$ | O | 4-$SCF_3$ | 76-78 |
| 53 | O | 3-Cl | i-$C_3H_7$ | $CH_2C_6H_5$ | O | 3-$CF_3$,4-Cl | Öl |
| 54 | O | 3-$CH_3$ | i-$C_3H_7$ | $CH_3$ | O | 4-$SCF_3$ | amorph |
| 55 | O | 3-Cl | $CH_3$ | $C_2H_5$ | O | 3-Cl,4-$CF_3$ | Öl |
| 56 | O | 3-Cl | $CH_3$ | $C_2H_5$ | O | 2,4-$(CF_3)_2$ | 97-99 |
| 57 | O | 3-Cl | $CH_3$ | $C_2H_5$ | O | 3-$CF_3$,4-Cl | amorph |
| 58 | O | 3-Cl | $CH_3$ | $C_2H_5$ | O | 4-$SCF_3$ | amorph |
| 59 | O | 3-Cl | $CH_3$ | $C_2H_5$ | O | 4-$OCF_3$ | Öl |
| 60 | O | 4-Cl | $C_6H_5$ | $CH_3$ | O | 4-$SCF_3$ | 161-2°C |
| 61 | O | 3-Cl | $CH_3$ | $C_2H_5$ | O | 3,4-$(CF_3)_2$ | Öl |
| 62 | O | 3-Cl | $CH_3$ | i-$C_3H_7$ | S | H | Öl |
| 63 | O | 3-Cl | $CH_3$ | i-$C_3H_7$ | O | 3,4(-$CF_2$-O-$CF_2$-O-) | Öl |
| 64 | O | 3-Cl | $CH_3$ | i-$C_3H_7$ | O | 3-Cl,4-$SCF_3$ | 154-5°C |
| 65 | O | 3-$CH_3$ | $CH_3$ | i-$C_3H_7$ | O | 3-$CF_3$,4Cl | amorph |
| 66 | O | H | 4-$C_6H_4Cl$ | $CH_3$ | O | 4-$SCF_3$ | 151-153° |

0216105

Herstellung der Ausgangsprodukte:
Allgemeine Vorschrift zur Herstellung der 1,3,2-Diazaphosphorine

0,3 Mol des Phosphorsäureesterdiamids werden in 500 ml trockenem Toluol gelöst und unter Stickstoff innerhalb von 40 Minuten 0,33 Mol Malonsäuredichlorid zugetropft. Während des Zutropfens wird die Reaktionsmischung vorsichtig auf 75°C erhitzt (kräftige Gasentwicklung).

Anschließend wird solange bei 80°C nachgerührt (üblicherweise 2-3 h), bis die HCl-Entwicklung aufhört und eine Probe nach dünnschichtchromatographischer Prüfung kein Ausgangsmaterial mehr enthält. Der Reaktionsansatz wird dann mit 250 ml Wasser verrührt, die wäßrige Phase und eventuell gebildetes Öl abgetrennt und die organische Phase mit Natriumhydrogencarbonat-Lösung neutral gewaschen.

Nach dem Trocknen über $Na_2SO_4$ wird das Toluol im Vakuum abdestilliert und der Rückstand umkristallisiert (Ethanol/Petrolether).

Analog werden die folgenden Verbindungen hergestellt:

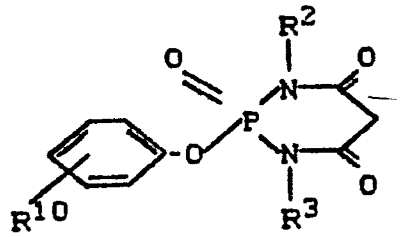

| Beispiel Nr. | $R^{10}$ | $R^2$ | $R^3$ | Fp/°C | umkristallisiert aus |
|---|---|---|---|---|---|
| a | 3-Cl | i-$C_3H_7$ | i-$C_3H_7$ | 85-85,5 | EtOH/PE |
| b | 3-$CH_3$ | i-$C_3H_7$ | i-$C_3H_7$ | 62-65 | EtOH/PE |
| c | 3-Cl | $C_2H_5$ | $CH_3$ | 54-55 | EtOH/PE |
| d | 3-Cl | $CH_3$ | i-$C_3H_7$ | 80-81 | EtOH/PE |
| e | 3-$CH_3$ | 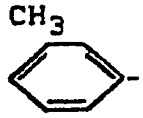-$CH_2$ | i-$C_3H_7$ | 82-83 | EtOH/PE |
| f | H | $CH_3$ | 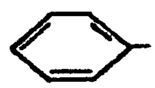 | 134-135 | EtOH/PE/Tol |
| g | 3-Cl | 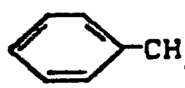-$CH_2$ | i-$C_3H_7$ | 91-93 | EtOH/PE |
| h | 3-$CH_3$ | $CH_3$ | i-$C_3H_7$ | 60-62 | EtOH/PE |
| i | 4-Cl | $CH_3$ | $CH_3$ | 163 | EtOH |
| k | 4-$CH_3$ | $CH_3$ | $CH_3$ | 78 | Ligroin |
| l | 3-$CH_3$ | 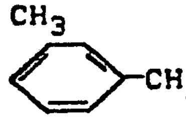-$CH_2$ | $CH_3$ | 86-88 | EtOH/PE |
| m | 3-Cl | 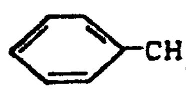-$CH_2$ | $CH_3$ | 94-96 | EtOH/PE |

## Patentansprüche:

1. Diazaphosphorine der Formel I

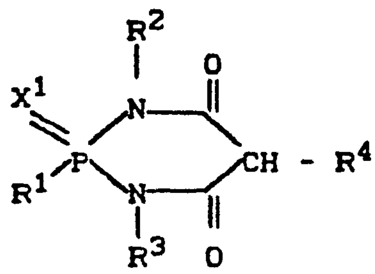

I

in welcher

$X^1$ für O oder S steht

$R^1$ für Aryloxy oder Arylthio steht,

$R^2$ für Wasserstoff, Alkyl, Aryl oder Aralkyl steht,

$R^3$ für Wasserstoff, Alkyl, Aryl oder Aralkyl steht,

$R^4$ für den Rest der Formel steht,

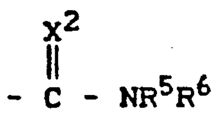

wobei

$X^2$ für O oder S steht,

$R^5$ für Wasserstoff oder Alkyl steht,

$R^6$ für den Fall, daß $X^1$ für O steht, für Phenyl steht, das mindestens einen Substituenten aus der Gruppe $NO_2$, Halogenalkyl, Alkoxy, Phenyloxy, das gegebenenfalls substituiert ist, Alkylthio, Phenylthio, das gegebenenfalls substituiert ist, Halogenalkoxy, Halogenalkylthio, Alkylendioxy, das gegebenenfalls halogensubstituiert ist, Alkylsulfenyl, Alkylsulfonyl, Halogenalkylsulfenyl, Halogenalkylsulfonyl, Halogensulfonyl ($HalSO_2$-) und gegebenenfalls noch weitere Substituenten aus der Gruppe Halogen, Alkyl enthält, unter der Voraussetzung, daß für den Fall, daß $R^6$ für einen Halogenalkylrest steht, noch weitere Substituenten vorhanden sind,

$R^6$ für den Fall, daß $X^2$ für S steht, für gegebenenfalls substituiertes Phenyl steht.

Die Verbindungen der Formel I können in Form ihrer verschiedenen Tautomeren (Keto/Enol) sowie als Gemische dieser Tautomeren, sowie in Form ihrer Salze mit Basen vorliegen.

2. Verfahren zur Herstellung der Verbindungen der Formel I

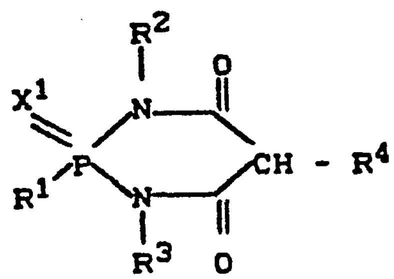

I

in welcher

$X^1$ für O oder S steht

$R^1$ für Aryloxy oder Arylthio steht,

$R^2$ für Wasserstoff, Alkyl, Aryl oder Aralkyl steht,

$R^3$ für Wasserstoff, Alkyl, Aryl oder Aralkyl steht,

$R^4$ für den Rest der Formel steht,

$$-\overset{\overset{X^2}{\|}}{C}-NR^5R^6$$

wobei

$X^2$ für O oder S steht,

$R^5$ für Wasserstoff oder Alkyl steht,

$R^6$ für den Fall, daß $X^1$ für O steht, für Phenyl steht, das mindestens einen Substituenten aus

der Gruppe $NO_2$, Halogenalkyl, Alkoxy, Phenyloxy, das gegebenenfalls substituiert ist, Alkylthio, Phenylthio, das gegebenenfalls substituiert ist, Halogenalkoxy, Halogenalkylthio, Alkylendioxy, das gegebenenfalls halogensubstituiert ist, Alkylsulfenyl, Alkylsulfonyl, Halogenalkylsulfenyl, Halogenalkylsulfonyl, Halogensulfonyl ($HalSO_2$-) und gegebenenfalls noch weitere Substituenten aus der Gruppe Halogen, Alkyl enthält, unter der Voraussetzung, daß für den Fall, daß $R^6$ für einen Halogenalkylrest steht, noch weitere Substituenten vorhanden sind,

$R^6$ für den Fall, daß $X^2$ für S steht, für gegebenenfalls substituiertes Phenyl steht,

dadurch gekennzeichnet, daß man

a) für den Fall, daß $R^5$ für Wasserstoff steht, Diazaphosphorine der Formel II

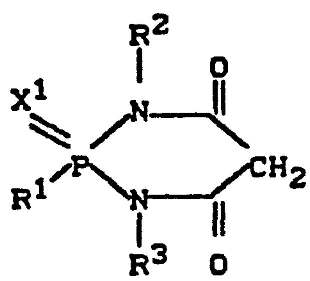

II

in welcher

$X^1$, $R^1$, $R^2$, $R^3$ die oben angegebene Bedeutung haben,

0216105

mit Isocyanaten der Formel III

$R^6$ - NCO(S) III

in welcher

$R^6$ die oben angegebene Bedeutung hat,

gegebenenfalls in Gegenwart von Katalysatoren umsetzt,

b) Diazaphosphorine der Formel IV

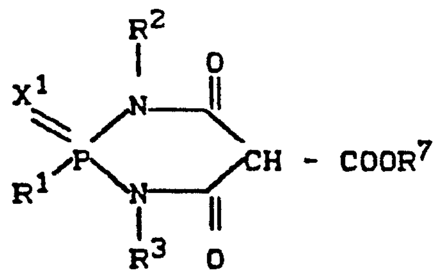

IV

in welcher

$X^1$, $R^1$, $R^2$, $R^3$ die oben angegebene Bedeutung haben,

$R^7$ für $C_1$-$C_4$-Alkyl steht,

mit Aminen der Formel V

$HNR^5R^6$ V

in welcher

$R^5$ und $R^6$ die oben angegebene Bedeutung haben,

umsetzt.

3. Schädlingsbekämpfungsmittel, gekennzeichnet durch einen Gehalt an mindestens einem Diazaphosphorin der Formel (I), gemäß Anspruch 1.

4. Verwendung von Diazaphosphorinen der Formel (I) gemäß Anspruch 1 zur Bekämpfung von Schädlingen.

5. Verfahren zur Herstellung von Schädlingsbekämpfungsmitteln, dadurch gekennzeichnet, daß man Diazaphosphorine der Formel (I) gemäß Anspruch 1 mit Streckmitteln und/oder oberflächenaktiven Mitteln vermischt.

6. Mittel zur Bekämpfung von Endoparasiten, gekennzeichnet durch einen Gehalt an mindestens einem Diazaphosphorin der Formel I gemäß Anspruch 1.

7. Verwendung von Diazaphosphorinen der Formel I gemäß Anspruch 1 zur Bekämpfung von Endoparasiten.

8. Verfahren zur Herstellung von endoparasitiziden Mitteln, dadurch gekennzeichnet, daß man Diazaphosphorine der Formel I gemäß Anspruch 1 verwendet.

Europäisches Patentamt

# EUROPÄISCHER RECHERCHENBERICHT

0216105

Nummer der Anmeldung

EP 86 11 1105

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| A | CHEMICAL ABSTRACTS, Band 97, Nr. 15, 11. Oktober 1982, Seite 736, rechte Spalte, Zusammenfassungsnr. 127733z, Columbus, Ohio, US; H.J. ROTH et al.: "Phosphorodiamidates. IV. Synthesis of new phosphorins", & ARCH. PHARM. (WEINHEIM, GER.) 1982, 315(7), 585-589<br>--- | 1 | C 07 F 9/65<br>A 01 N 57/36<br>A 61 K 31/675 |
| X,D | CH-A- 643 563 (CIBA-GEIGY)<br>* Ansprüche 1, 8 *<br>--- | 1,2 | |
| A,D | DE-A-2 600 665 (H.J. ROTH et al.)<br>----- | 1 | |
| | | | **RECHERCHIERTE SACHGEBIETE (Int Cl 4)** |
| | | | C 07 F 9/65<br>A 01 N 57/36 |

Der vorliegende Recherchenbericht wurde für alle Patentansprüche erstellt

| Recherchenort | Abschlußdatum der Recherche | Prüfer |
|---|---|---|
| BERLIN | 18-11-1986 | KAPTEYN H G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82